# EUROPEAN PATENT APPLICATION

(11) **EP 1 849 463 A2**
(43) Date of publication of application: **31.10.2007**
(21) Application number: 06012442.7
(22) Date of filing: 16.06.2006
(51) Int. Cl.: A61K 9/70, A61K 31/167, A61K 31/7036, A61K 38/14, A61K 38/48

(54) **Microbial cellulose materials for use in transdermal drug delivery systems, methods of manufacture and use**

(30) Priority: 19.04.2006 US 406528
(71) Applicant: Xylos Corporation, Langhorne, PA 19047 (US)
(72) Inventor: Serafica, Gonzalo, Langhorne, PA 19047 (US); Mink, Richard, Yardley, PA 19067 (US); Hoon, Russell, Doylestown, PA 18901 (US); Damien, Christopher, Newton, PA 18940 (US)
(74) Representative: HOFFMANN EITLE

(57) **Abstract**

The invention provides a transdermal delivery system comprising microbial cellulose, water, and a therapeutically effective amount of a biologically active agent, as well as the use of microbial cellulose for the manufacture of a transdermal drug delivery system. The transdermal delivery system can achieve the delivery of a drug at a substantially constant rates over extended periods.

## Description

### FIELD OF INVENTION

This invention relates to polysaccharide materials and more particularly to microbial cellulose for use in transdermal drug delivery systems. The invention also relates to the use of microbial cellulose and microbial cellulose composites containing various agents as transdermal drug delivery devices and to the methods of producing and using such materials.

### BACKGROUND

One of the functions of skin is to protect the body from aqueous, biological, chemical, and mechanical assault. To achieve this, the outermost layer, called the epidermis, ranges from 75 to 600 microns in thickness and contains cells that limit the permeability of various low and high molecular weight active agents. It is also avascular such that any active agent must pass through the skin to reach the dermis before it can enter the bloodstream and have a systemic effect.

Various physical and chemical means have been used to enhance delivery of drugs and active agents by creating holes through the epidermis or affecting the skin's lipid bilayer to allow agents to pass. Physical methods such as microporation, electroporation and sonophoresis have been actively investigated (Chiarello, K., Breaking the Barrier, Pharmaceutical Technology, October 2004). Microporation is the method whereby very small needles (micro-needles) physically produce channels through the epidermis into the dermal and fat layers. Electroporation uses a limited electric field of 100 volts or more to create aqueous pores in the lipid bilayer through which agents can be delivered. This technique is described in U.S. Patent 5,019,034. Sonoporation follows the same premise but uses sound waves in conjunction with a gel-like material to disrupt the epidermal layer and allow agents to pass. This is described in U.S. Patent 4,767,402 and more recently in 6,487,447.

Iontophoresis is a method different from the previous whereby an ionic transfer is created by placing a low voltage electric field to 'ionize' agents and allow them to pass through the skin by an ionic gradient. This method is described in U.S. Patent Number 5,224,927 and 5,540,669.

Chemical enhancers such as hydroxide donors and polyols have been reported. U.S. Patent 6,673,363 describes the use of basic and hydroxide-releasing agents as chemical enhancers to increase the permeation rate of various types of active agents. The use of monoalkyl phosphates has also been employed by another group in U.S Patent 5,308,625 to increase the permeability of active agents through the top layer of the epidermis (stratum corneum). Multiphase systems of water and oil have also been described in U.S. Patent 6,299,902 where the two phases enhance the delivery of anesthetic agents.

However, none of the methods previously described suggests creating a highly hydrated environment by using hydrated microbial cellulose sheets to deliver drugs and active agents. Also, the use of microbial cellulose sheets (containing as much as 99% water) in contact with intact skin for extended periods to promote active agent delivery has not been disclosed. The ability to use a material containing high levels of water that is not bound (water activity of 1) to enhance the permeability of skin has not been disclosed as well. The idea of increasing the number of pores, reducing pore tortuosity or creating water channels through intact skin has been theorized by exposure of pig skin to water for 24 to 48 hours and by using outside forces such as electroporation and sonoporation. However, the ability of highly hydrated sheets of microbial cellulose to promote the creation of such aqueous channels, pores or reduced tortuosity on intact skin has not been disclosed previously. Likewise the combination of promoting pore changes by electroporation or other methods and supplying the drug or active agent via a highly hydrated microbial cellulose device has not been envisioned previously.

Microbial cellulose (MC) can be used as a transdermal drug delivery device (TDDD) due to its ability to continuously hydrate the intact skin's stratum *corneum;* thereby creating channels in the skin that can allow increased permeation of active agents into and through intact skin. MC sheets having up to 99% water can used to hydrate skin and the active agents can be suspended in the water contained in the sheets. Physical and chemical permeation enhancers can be used in conjunction with microbial cellulose sheets to effect the desired level of delivery. Physical methods such as electric (iontophoresis and electroporation), ultrasound (sonophoresis), microporation, heat and pressure (jet injectors) can be utilized along with the use of MC TDD. Additional materials such as polyols, surfactants, liposomes, hydroxide donors and solubility enhancers may also be used in conjunction with hydrated microbial cellulose to increase active agent solubility or increase skin permeability via chemical means.

### SUMMARY OF THE INVENTION

There is provided, a new class of transdermal drug and active agent delivery materials utilizing microbial cellulose for use in a wide variety of topical applications including for intact and breached skin.

There is provided, in accordance with another preferred embodiment of the invention, methods of applying microbial cellulose in a wide variety of applications that utilize the desirable physical and chemical properties of microbial cellulose.

There is provided, in accordance with another preferred embodiment of the invention, a process for the preparation of these aforementioned materials that will yield the desirable properties for each particular product application.

There is provided, in accordance with another preferred embodiment of the invention, the use of microbial cellulose for the manufacture of a transdermal drug delivery system.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description. The invention, thus generally described, will be understood more readily by reference to the following examples, which are provided by way of illustration and are not intended to be limiting of the present invention.

### DETAILED DESCRIPTION OF THE PREFERRED EMBODIMENTS

According to one preferred embodiment, the present invention provides a transdermal delivery system comprising microbial cellulose, water, and a biologically active agent. The biologically active agent can be administered to a subject in need thereof by topically applying a composition comprising the insoluble microbial cellulose, water, and a therapeutically effective amount of the biologically active agent. The biologically active agent generally penetrates through the subject's stratum *corneum* at a substantially constant rate. The skin penetration rate can be determined in vitro using human cadaver skin. Preferably, the microbial cellulose is produced by Acetobacter *xylinum.* The microbial cellulose is preferably hydrated with up to 99% water by weight of the composition, more preferably, with 75% to 99% water by weight of the composition. In other words, the water content is preferably up to 99 wt.%, more preferably 75 to 99 wt.% with respect to the total weight of microbial cellulose and water (i.e. without further ingredients such as the biologically active agent). In a particularly preferred embodiment, the transdermal delivery system contains 1.5 to 9 wt.%, more preferably 3 to 6 wt.%, and most preferred 3.4 to 4.5 wt.% microbial cellulose with respect to the total weight of the transdermal delivery system (without occlusive backing). Preferred biologically active agents are drugs, including an antibiotic, antifungal or hemostatic agent. Preferred antibiotics include vancomycin, tobramycin, or neomycin. Preferred antifungals are voriconazole, clotrimazole, or miconazole. A preferred hemostatic agent is thrombin. Also preferred is an anesthetic biologically active agent, such as lidocaine. Other preferred drugs are fentanyl and insulin. The biologically active agent is preferably present in an amount so that it is capable of penetrating through human cadaver skin at a substantially constant rate. "Substantially constant rate" means that the flux of the agent through the skin is always within +/- 20 %, preferably +/- 10 % of the average skin flux. The rate is preferably substantially constant over at least 2 hours, more preferably at least 6 hours, and most preferably at least 10 hours. The amount of biologically active agent necessary to achieve this substantially constant rate generally varies within 1 to 30 wt.%, more preferably 2 to 10 wt.% of the total weight of the transdermal delivery system. For example, the biologically active agent can be incorporated in the microbial cellulose sheet by soaking the microbial cellulose sheet in a solution of 1 to 30 wt.%, more preferably 2 to 10 wt.% of the biologically active agent , e.g. by soaking in an initial solution of 2-10% lidocaine by weight of the solution. Also in a preferred embodiment, the biologically active agent penetrates through the stratum corneum in less than one hour, even more preferably, in less than one-half an hour. In other words, within this time, the biologically active agent is administered in a therapeutically active amount. The delivery rate (flux rate) may drop afterwards, i.e. after one hour, more preferably after one-half an hour. Due to its ability to continuously hydrate the stratum corneum, the transdermal drug delivery device of the present invention does not require additional components to enhance delivery of the biologically active agent. Hence, in a particularly preferred embodiment, the device essentially consists of microbial cellulose, water, and the biologically active agent. However, the composition may optionally include at least one additional component to enhance delivery of the biologically active agent selected from the group consisting of an electroporation component, a microporation component, a sonophoresis component, and an iontophoresis component. According to the invention, the composition can be topically applied to intact skin or breached skin to deliver the biologically active agent through the stratum corneum. An additional preferred embodiment is a method wherein the composition is covered by an occlusive backing. A preferred wound dressing formulation of 96% water and 4% insoluble microbial cellulose has been shown to effectively deliver drugs such as antibiotics and lidocaine through breached skin and chronic wounds into surrounding areas of stratum corneum to reduce pain and prevent infection.

The use of MC on both breached and intact skin to deliver beneficial components has distinct advantages of using water to enhance the delivery by increasing skin permeability. The creation of changes in the pores of highly hydrated intact skin may be a safe and efficient means to conduct transdermal drug delivery of a variety of drugs and pharmaceutical agents both small (e.g., antibiotics) and large molecules (e.g., insulin).

Drug delivery products wherein microbial cellulose sheets are loaded with water, chemical enhancers and active agents or drugs used alone or in conjunction with other drug delivery methods are envisioned.

In a preferred embodiment, the invention includes a topical drug delivery system comprising microbial cellulose of the instant invention and a drug or other active agent. The invention also provides a method of drug delivery, comprising providing a topical composition comprising microbial cellulose and drug and applying said composition on the subject in need thereof.

Exemplary active drugs that can be administered by the novel transdermal drug delivery system of this invention include, but are not limited to:

Cardioactive medications, illustratively, organic nitrates such as nitroglycerin, isosorbide dinitrate and isosorbide mononitrates; quinidine sulfate; procainamide; thiazides such as bendroflumethiazide, chlorothiazide, and hydrochlorothyazide; nifedipine; nicardipine; adrenergic blocking agents, such as timolol, and propranolol; verapamil; diltiazem; captopril; clonidine and prazosin and the like.

Androgenic steroids, such as, testosterone; methyltestosterone; and fluoxymesterone and the like.

Estrogens, such as conjugated estrogens, esterified estrogens, estropipate, 17.beta.-estradiol, 17.beta.- estradiol valerate, equilin, mestranol, estrone, estriol, 17.beta.-ethinyl estradiol, and diethylstilbestrol and the like.

Progestational agents, such as progesterone, 19-norprogesterone, norethindrone, norethindrone acetate, melengestrol, chlormadinone, ethisterone, medroxyprogesterone acetate, hydroxyprogesterone caproate, ethynodiol diacetate, norethynodrel, 17-alpha-hydroxyprogesterone, dydrogesterone, dimethisterone, ethinylestrenol, norgestrel, demegestone, promegestone, and megestrol acetate and the like.

Drugs having an action on the central nervous system, for example sedatives, hyponotics, antianxiety agents, analgesics and anesthetics, such as chloral, buprenorphine, naloxone, haloperidol, fluphenazine, pentobarbital, phenobarbital, secobarbital, codeine, lidocaine, tetracaine, dyclonine, dibucaine, cocaine, procaine, mepivacaine, bupivacaine, etidocaine, prilocaine, benzocaine, fentanyl, and nicotine and the like.

Nutritional agents, such as vitamins, essential amino acids and essential fats and the like.

Anti-inflammatory agents, such as hyd rocortisone, cortisone, dexamethasone, fluocinolone, triamcinolone, medrysone, prednisolone, flurandrenolide, prednisone, halcinonide, methylprednisolone, prednisone, halcinonide, methylprednisolone, fludrocortisone, corticosterone, paramethasone, betamethasone, ibuprophen, naproxen, fenoprofen, fenbufen, flurbiprofen, indoprofen, ketoprofen, suprofen, indomethacin, piroxicam, aspirin, salicylic acid, diflunisal, methyl salicylate, phenylbutazone, sulindac, mefenamic acid, meclofenamate sodium, tolmetin, and the like.

Antihistamines, such as diphenhydramine, dimenhydrinate, perphenazine, triprolidine, pyrilamine, chlorcyclizine, promethazine, carbinoxamine, tripelennamine, brompheniramine, hydroxyzine, cyclizine, meclizine, clorprenaline, terfenadine, and chlorpheniramine and the like.

Respiratory agents, such as theophilline and beta. sub.2 -adrenergic agonists such as albuterol, terbutaline, metaproterenol, ritodrine, carbuterol, fenoterol, quinterenol, rimiterol, solmefamol, soterenol, tretoquinol and the like.

Sympathomimetics such as dopamine, norepinephrine, phenylpropanolamine, phenylephrine, pseudoephedrine, amphetamine, propylhexedrine, epinephrine and the like.

Miotics, such as pilocarpine, and the like.

Cholinergic agonists, such as choline, acetylcholine, methacholine, carbachol, bethanechol, pilocarpine, muscarine, and arecoline and the like.

Antimuscarinic or muscarinic cholinergic blocking agents, such as atropine, scopolamine, homatropine, methscopolarnine, homatropine methylbromide, methantheline, cyclopentolate, tropicamide, propantheline, anisotropine, dicyclomine, eucatropine and the like.

Mydriatics, such as atropine, cyclopentolate, homatropine, scopolamine, tropicamide, eucatropine, hydroxyamphetamine and the like.

Psychic energizers, such as 3-(2-aminopropy) indole, 3-(2-aminobutyl)indole, and the like.

Anti-infectives, such as antibiotics, including penicillin, tetracycline, chloramphenicol, sulfacetamide, sulfamethazine, sulfadiazine, sulfamerazine, sulfamethizole and sulfisoxazole; antivirals, including idoxuridine; antibacterials, such as erythromycin and clarithromycin; and other anti-infectives including nitrofurazone and the like.

Humoral agents, such as the prostaglandins, natural and synthetic, for example PGE1, PGE 2-alpha, and PGF 2-alpha, the PGE1 analog misoprostol and the like.

Antispasmodics, such as atropine, methantheline, papaverine, cinnamedrine, methscopolamine and the like.

Antidepressant drugs, such as isocarboxazid, phenelzine, tranylcyprornine, imipramine, amitriptyline, trimipramine, doxepin, desipramine, nortriptyline, protriptyline, amoxapine, maprotiline, trazodone and the like.

Anti-diabetics, such as insulin, and anticancer drugs such as tamoxifen, methotrexate and the like.

Anorectic drugs, such as, dextroamphetamine, methamphetamine, phenylpropanolamine, fenfluramine, diethylpropion, mazindol, phentermine and the like.

Anti-allergenics, such as antazoline, methapyrilene, chlorpheniramine, pyrilamine, pheniramine and the like.

Tranquilizers, such as reserpine, chlorpromazine, and antianxiety benzo-diazepines such as alprazolam, chlordiazepoxide, clorazeptate, halazepam, oxazepam, prazepam, clonazepam, flurazepam, triazolam, lorazepam, diazepam and the like.

Antipsychotics, such as thiopropazate, chlorpromazine, triflupromazine, mesoridazine, piperacetazine, thioridazine, acetophenazine, fluphenazine, perphenazine, trifluoperazine, chlorprothixene, thiothixene, haloperidol, bromperidol, loxapine, molindone and the like.

Decongestants, such as phenylephrine, ephedrine, naphazoline, tetrahydrozoline and the like.

Antipyretics, such as aspirin, salicylamide, and the like.

Anti-migraine agents, such as dihydroergotarnine, pizotyline and the like.

Drugs for treating nausea and vomiting, such as chlorpromazine, perphenazine, prochlorperazine, promethazine, triethylperazine, triflupromazine, and trirneprazine and the like.

Anti-malarials, such as the 4-aminoquinolines, alphaamino-quinolines, chloroquine, pyrimethamine and the like.

Anti-ulcerative agents, such as misoprostol, omeprazole, enprostil and the like.

Peptides, such as growth releasing factor and the like.

Drugs for Parkinson's disease, spasticity, and acute muscle spasms such as levodopa, carbidopa, amantadine, apomorphine, bromocriptine, selegiline (deprenyl), trihexyphenidyl hydrochloride, benztropine mesylate, procyclidine hydrochloride, baclofen, diazepam, dantrolene and the like.

Anti-estrogen or hormone agents, such as tamoxifen or human chorionic gonadotropin and the like.

The drug can be in its free base or acid form, or in the form of salts, esters, or any other pharmacologically acceptable derivatives, enantomerically pure forms, tautomers or as components of molecular complexes. The amount of drug to be incorporated in the composition varies depending on the particular drug, the desired therapeutic effect, and the time span for which the device is to provide therapy. Generally, for purposes of the invention, the amount of drug in the system can vary from about 0.0001% to as much as 60%. The invention is further illustrated by, though in no way limited to the following examples.

### Example 1

### Delivery of antibiotics

In cases of difficult to heal wounds, clinicians believe that there may be microbial colonization that has not reached levels of infection. Depending on the type of microbe (gram negative or gram positive bacteria, fungus, etc) a specific treatment is optimal. Patients having wounds that were no longer responsive to treatment were evaluated for colonization and split into various groups. Those with gram-positive bacteria were treated with microbial cellulose to which intravenous (IV) vancomycin was added. Those with gram-negative bacteria were treated with IV tobramycin-soaked microbial cellulose. Patients with fungal growth were treated with an antifungal. Dressings were changed as needed. Results indicated that the vancomycin treated wounds improved and went on to heal in an average of five weeks. Serum trough levels of the antibiotics (values of the amount of an antibiotic in the blood) demonstrated a more profound local effect than that which passed into the circulatory system. This demonstrated the delivery of antibiotics by microbial cellulose.

### Example 2

### Delivery of lidocaine by Microbial Cellulose

A solution of 4% lidocaine was added to microbial cellulose and allowed to soak into the microbial cellulose for 5 minutes. This was then placed onto the patient's wound for 5 to 15 minutes prior to sharp debridement of necrotic tissue. Delivery of the lidocaine was evidenced by the lack of pain that the patient felt during the debridement process. A similar study performed on intact skin demonstrated that lidocaine was released over a 7-day period to relieve pain administered by a sharp object.

### Example 3

### Delivery of Thrombin by Microbial Cellulose

Thrombin was reconstituted per instructions for use and applied to microbial cellulose for 5 minutes. The dressing containing the thrombin solution was applied to a patient that had excessive diffuse bleeding after sharp debridement and was removed after 24 hours. The combination of thrombin and microbial cellulose was very effective at reducing/stopping bleeding.

### Example 4

### Transdermal delivery of Lidocaine by microbial cellulose in vitro using human cadaver skin diffusion cell study

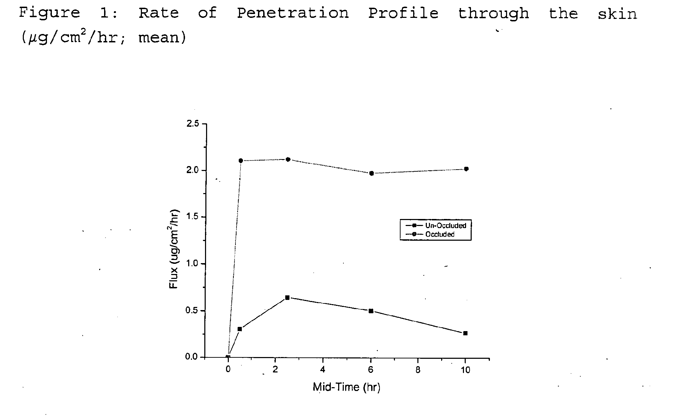

The study was carried out to test microbial cellulose containing a solution of 10% lidocaine HCl in water tested on two different skin donors using a finite dose in vitro cadaver skin model. The initial cellulose concentration is about 4% cellulose prior to soaking in a 25% lidocaine solution to arrive at the desired 10% in the dressing. The study was done using 1-cm square patches of skin performed in duplicate. The test articles were tested in both occluded and non occluded chambers to compare their performance. In all instances, the human cadaver trunk skin was mounted onto Franz diffusion cells and the microbial cellulose with 10% lidocaine HCl was placed on one side of the cell for dosing. Reservoir solutions were collected at pre-selected time intervals. After the last sample was collected, the surface of the skin was washed and the epidermis and dermis was isolated and retained for analysis. The samples collected were processed and analyzed using High Performance Liquid Chromatography (HPLC) for lidocaine content. The results are presented in Table 1-2 and Figure 1. The results indicate higher transdermal penetration with microbial cellulose with 10% lidocaine HCl in the occluded chamber. Also results demonstrate that a fast delivery is achieved in both samples and a sustained delivery of the active agent is more pronounced in the occluded chamber as compared to the samples that are allowed to dehydrate during the test (non occluded). The conclusions derived from the study are the following: 1) the formulation of microbial cellulose containing 10% lidocaine HCl without any additional chemical enhancer or active driving force (i.e., iontoporetic) has been shown to penetrate the *stratum corneum* and deliver the active ingredient into and through the epidermis and dermis and 2) the delivery is fast and sustained at a steady state in the occluded chamber tests.

**Table 1: Rate of Penetration Profile through the skin (µg/cm²/hr; mean ± se)**

| **Sample** | **Un-Occluded** | **Occluded** | **Mid-Time** (hr) |
|---|---|---|---|
| 1 | 0.307 ± 0.307 | 2.106 ± 1.828 | 0.5 |
| 2 | 0.645 ± 0.162 | 2.123 ± 2.123 | 2.5 |
| 3 | 0.506 ± 0.376 | 1.977 ± 1.909 | 6 |
| 4 | 0.270 ± 0.270 | 2.027 ± 1.876 | 10 |

| | | | |
|---|---|---|---|
| Mid-time is the central point between sample collections. | | | |

**Table 2: Distribution of Applied Dose (micrograms/cm²; mean ± se)**

| Sample Source | Un-Occluded | Occluded |
|---|---|---|
| Total Absorbed | 5.35 ± 2.76 | 24.49 ± 23.34 |
| Dermis | 0.21 ± 0.21 | 2.29 ± 0.65 |
| Epidermis | 5.37 ± 0.80 | 16.87 ± 7.99 |

## Claims

1. A transdermal delivery system comprising microbial cellulose, water, and a therapeutically effective amount of a biologically active agent.

2. The transdermal delivery system of claim 1, wherein the biologically active agent is present in an amount so that it is capable of penetrating through human cadaver skin at a substantially constant rate.

3. The transdermal delivery system of claim 1 or 2, wherein the microbial cellulose is produced by Acetobacter *xylinum.*

4. The transdermal delivery system of any one of the preceding claims, wherein the microbial cellulose is hydrated with up to 99% water by weight of the composition.

5. The transdermal delivery system of claim 4, wherein the microbial cellulose is hydrated with 75% to 99% water by weight of the composition.

6. The transdermal delivery system of claim 5, wherein the biologically active agent is an antibiotic, antifungal or hemostatic agent, or an anesthetic.

7. The transdermal delivery system of claim 6, wherein the antibiotic is vancomycin, tobramycin, or neomycin.

8. The transdermal delivery system of claim 6, wherein the antifungal is voriconazole, clotrimazole, or miconazole.

9. The transdermal delivery system of claim 6, wherein the hemostatic agent is thrombin.

10. The transdermal delivery system of claim 6, wherein the anesthetic is lidocaine.

11. The transdermal delivery system of claim 10, wherein the composition is loaded with lidocaine by soaking in an initial solution of 2-10% lidocaine by weight of the solution.

12. The transdermal delivery system of any one of claims 1 to 5, wherein the biologically active agent is fentanyl.

13. The transdermal delivery system any one of claims 1 to 5, wherein the biologically active agent is insulin.

14. The transdermal delivery system of any one of the preceding claims, wherein the system is capable of continuously delivering a therapeutically effective amount of the biologically active agent at a substantially constant rate for at least 10 hours through human cadaver skin.

15. The transdermal delivery system of any one of claims 1 to 13, wherein the biologically active agent penetrates through through human cadaver skin in less than one hour.

16. The transdermal delivery system of any one of claims 1 to 13, wherein the biologically active agent penetrates through the stratum corneum is less than one-half of an hour.

17. The transdermal delivery system of any one of the preceding claims, wherein the composition includes at least one additional component to enhance delivery of the biologically active agent selected from the group consisting of an electroporation component, a microporation component, a sonophoresis component, and an iontophoresis component.

18. The transdermal delivery system of any one of claims 1 to 17, wherein the composition contains no additional component to enhance delivery of the biologically active agent.

19. The transdermal delivery system of any one of the preceding claims, wherein the composition is covered by an occlusive backing.

20. The transdermal delivery system of any one of the preceding claims, wherein the biologically active agent is present in an amount of 2 to 10 wt.% with respect to the total weight of the system.

21. A use of microbial cellulose for the manufacture of a transdermal drug delivery system.
